**Europäisches Patentamt**

**European Patent Office**

**Office européen des brevets**

(19)

(11) Veröffentlichungsnummer: **0 196 646**
**B1**

(12) **EUROPÄISCHE PATENTSCHRIFT**

(45) Veröffentlichungstag der Patentschrift: **27.06.90**

(21) Anmeldenummer: **86104375.0**

(22) Anmeldetag: **29.03.86**

(51) Int. Cl.⁵: **A 61 B 17/12**

(54) **Abschnürvorrichtung für Körperteile.**

(30) Priorität: **30.03.85 DE 8509593 u**

(43) Veröffentlichungstag der Anmeldung:
**08.10.86 Patentblatt 86/41**

(45) Bekanntmachung des Hinweises auf die
Patenterteilung:
**27.06.90 Patentblatt 90/26**

(84) Benannte Vertragsstaaten:
**DE FR GB IT**

(56) Entgegenhaltungen:
**DE-A-2 824 037**
**DE-A-3 104 382**
**FR-A-2 321 264**

(73) Patentinhaber: **HOLTSCH**
**Metallwarenherstellung Maria Holtsch**
**In den Faltern 13**
**D-6204 Taunusstein 4 (DE)**

(72) Erfinder: **Holtsch, Peter E.**
**In den Faltern 13**
**D-6204 Taunusstein 4 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Abschnürvorrichtung für Körperteile, insbesondere für den Oberarm, beispielsweise zur notwendigen Blutstauung für Injektionen oder Blutentnahmen.

Es sind Vorrichtungen bekannt, bei denen ein Band durch ein Verschlußgehäuse geführt wird, das mittels einer Wippe durch entgegengesetzten Zug einer Schlinge festgeklemmt wird. Durch einen Druck auf den der Bandschlinge zugewendeten Schenkel der Wippe wird das Band wieder freigegeben, so daß sich die Schlinge vergrößern kann, siehe DE—A—28 24 037. In einigen bekannten Vorrichtungen bildet der der Schlinge zugewendete Schenkel der Wippe gleichzeitig den Bandverschluß, sei es als Einrastkante, Widerhaken od. dgl.

Diese Vorrichtungen haben den Nachteil, daß das Freigeben des Bandes, und damit das Vergrößern der Schlinge, nur durch Druck an einer bestimmten Stelle erfolgen kann, nämlich auf den der Schlinge zugewendeten Schenkel der Wippe. Je weiter von Schenkelende und näher dem Drehpunkt der Drucke erfolgt, um so schwerer ist das Lösen. Übt man den Druck über dem Drehpunkt der Wippe aus, so erfolgt keinerlei Wirkung, das Band wird nicht freigegeben.

Ein weiterer Nachteil bekannter Abschnürvorrichtungen ist, daß das Öffnen der Schlinge durch Druck auf die Einrasttaste in Richtung des Körperteils erfolgt und ein zweiter Handgriff zum Auffangen des gelösten Bandes erforderlich ist.

Es sind ferner Abschnürvorrichtungen bekanntgeworden, bei denen in einem Schloßgehäuse ein Klemmhebel schwenkbar gelagert ist, der unter der Wirkung einer Feder steht. Die Klemmung des Bandes erfogt mit dem Ende des Klemmhebels, welcher das Band gegen die untere Wand des Schloßgehäuses drückt. Anstelle der Feder kann auch ein anderes, elastisches Element, beispielsweise ein Gummistopfen, vorgesehen sein. Auch für diese bekannten Vorrichtungen gilt das oben Gesagte. Außerdem muß der Druck auf den Klemmhebel immer gegen den Druck der Feder erfolgen, wodurch das Lösen des Bandes mitunter schwierig ist.

Der vorliegenden Erfindung liegt die Aufgabe zugrunde, eine Abschnürvorrichtung der eingangs genannten Art zu schaffen, welche die oben beschriebenen Nachteile der bekannten Vorrichtungen nicht aufweist und welch außerdem den Vorteil der leichten und problemlosen Bedienung mit sich bringt.

Zur Lösung der gestellten Aufgabe wird die aus DE—A—28 24 037 bekannte Abschnürvorrichtung so ausgebildet wie im zweiten Teil des Patentanspruchs 1 definitiv.

Mit der Abschnürvrrichtung gemäß der erfindung wird der wesentliche Vorteil erreicht, daß das Band durch Druck auf einen beliebigen Punkt der Drucktaste freigegeben un die Schlinge dadurch vergrößert werden kann. Die Klemmwirkung entsteht dadurch, daß die Unterkante des Verschlußgehäuses und der Einrastarm gespreizt werden, da die Klemmwippe Druck über die beiden Drehpunkte gegen den Gehäuseboden ausübt. Dadurch entfällt auch, wie bei den bekannten Vorrichtungen erforderlich, das zusätzliche Federelement, um die Wippe gegen das Band zu drücken.

Gemäß einer besonderen Ausführungsform weist bei der Abschnürvorrichtung gemäß der Erfindung die Taste zwei abgewinkelte Seitenwände mit eine Aussparung am Drehpunkt der Klemmwippe und einen nach vorne springenden Ansatz für die im Gehäuse gelagerte Drehachse auf.

Eine weitere Ausführungsform der Abschnürvorrichtung gemäß der Erfindung besteht darin, daß die Wippe einen geraden Boden mit Seitenwänden zur Aufnahme der Drehachse im Gehäuse und zur Drehverbindung mit der Taste aufweist und am hinteren Ende mit einer nach oben gerichteten Stirnwand versehen ist.

Eine weitere Ausbildungsform der Abschnürvorrichtung gemäß der Erfindung besteht darin, daß der Einrastarm am vorderen Ende der Taste gabelförmig ausgebildet ist und zwei Haken zum Einrasten einer durch zwei seitliche Druckknöpfe betätigbaren Kappe aufweist.

Anhand der Zeichnungen soll am Beispiel einer bevorzugten Ausführungsform die Abschnürvorrichtung gemäß der Erfindung näher erläutert werden.

In den Zeichnungen zeigt

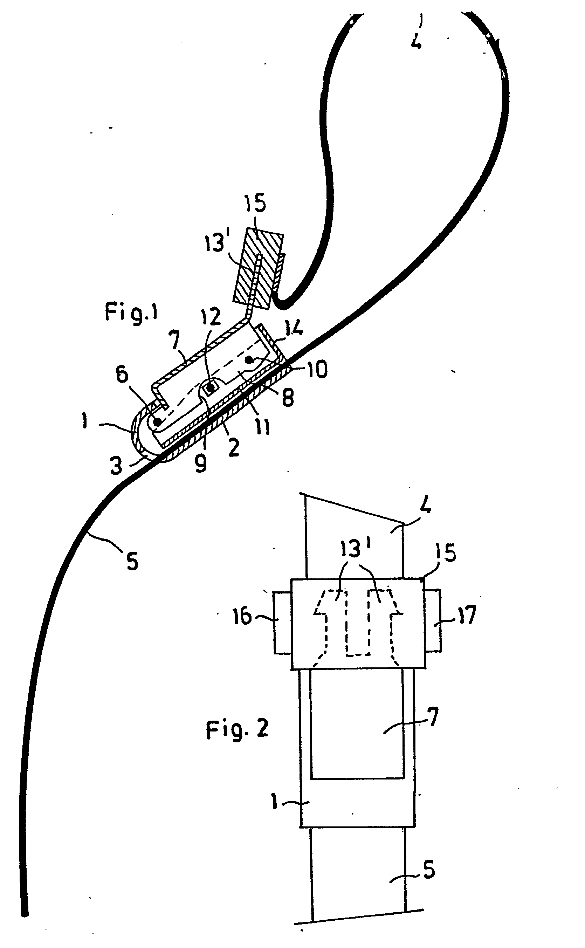

Fig. 1 eine Seitenansicht mit Teilaufriß der Abschnürvorrichtung gemäß der Erfindung.

Fig. 2 zeigt eine Draufsicht auf die Vorrichtung gemäß Fig. 1.

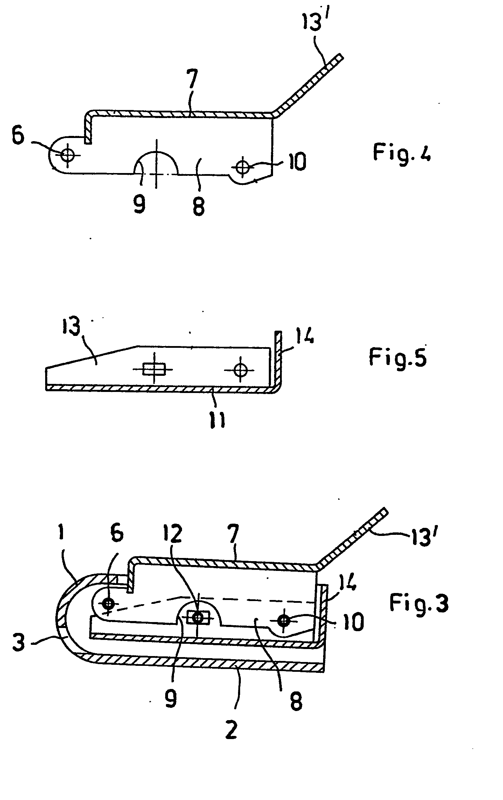

Fig. 3 zeigt einen Schnitt durch das Verschlußgehäuse.

Fig. 4 zeigt eine Seitenansicht der Taste.

Fig. 5 zeigt eine Seitenansicht der Klemmwippe.

Wie sich aus den Figuren der Zeichnungen ergibt, besteht die Abschnürvorrichtung gemäß der Erfindung aus einem Verschlußgehäuse 1 mit einem Boden 2 und einer vorderen, schlitzartigen Öffnung 3, durch die das eine Schleife 4 bildende Gummiband 5 verläuft. Im vorderen Teil des Gehäuses 1 ist um eine Achse 6 eine Taste 7 drehbar gelagert. Die Taste 7 weist Seitenwände 8 auf, die mit einer Aussparung 9 versehen sind. Am vorderen Ende der Seitenwände 8 ist um eine Achse 10 die Klemmwippe 11 drehbar gelagert. Die Klemmwippe 11 ist ihrerseits um eine mit dem Gehäuse 1 fest verbundene Achse 12 drehbar gelagert. Die Klemmwippe 11 weist ebenfalls Seitenwände 13 und eine hintere, nach oben gezogene Stirnwand 14 auf.

Am vorderen Ende weist die Tastatur 7 eine aus zwei gabelförmigen Ansätzen 13' gebildeten, schräg nach oben verlaufenden Einrastarm auf, auf dem die mit dem Ende des Bandes 5 verbundene Kappe 15 durch Einrasten festgeklemmt ist. Die Einrastung kann durch die beiden Druckknöpfe 16, 17 gelöst und so das Bandende freigegeben werden.

Zur Benutzung wird die Schlinge 4 um den entsprechenden, abzuschnürenden Körperteil gelegt und das Band 5 festgezogen. Die Klemmwirkung entsteht dadurch, daß die Unterkante des Gehäuses 1 und der Einrastarm 13′ gespreizt werden. Dadurch wird auf die Klemmwippe 11 ein Zug über die Drehpunkte 6, 10 ausgeübt und die Klemmwippe gegen den Boden des Gehäuses gedrückt, wodurch das Band 5 festgelegt wird.

Zum Lösen des Bandes kann auf jeden beliebigen Punkt der Taste 7 gedrückt werden, wodurch der Zug entlastet und die Druckwirkung der Klemmwippe auf das Band aufgehoben wird. Gleichzeitig kann zur schnellen Entfernung durch Betätigen der beiden Druckknöpfe 16, 17 das vordere, eine Ende des Bandes freigegeben werden.

## Patentansprüche

1. Abschnürvorrichtung für Körperteile, insbesondere für den Oberarm, beispielsweise zur notwendigen Blutstauung für Injektionen oder zur Blutentnahme, mit einem Verschlußgehäuse (1) zum Durchzug eines eine Schleife (4) bildenden Bandes (5) und einer im Verschlußgehäuse (1) angeordneten Klemmwippe (11) zum Festklemmen und Freigeben des Bandes, die etwa in der Mitte des Verschlußgehäuses (1) an einer mit dem Gehäuse fest verbundenen ersten Drehachse (12) drehbar gelagert ist, sowie mit Mitteln zur lösbaren Befestigung eines Bandendes am Verschluß, dadurch gekennzeichnet, daß im vorderen, der Schleife (4) abgewandten Bereich des Verschlußgehäuses (1) eine zweite Drehachse (6) vorgesehen ist, an der eine Drucktaste (7) dreshbar gelagert ist, die am entgegengesetzten Ende eine dritte Drehachse (10) aufweist, über die sie in drehbarer Verbindung mit der Klemmwippe (11) steht und daß die Drucktaste (7) am entgegengesetzten Ende einen schräg vom Gehäuse weg gereichteten Einrastarm (13′) zur lösbaren Befestigung des einen Bandendes aufweist.

2. Abschnürvorrichtung nach Anspruch 1, dadurch gekennzeichnet, daß die Taste (7) zwei abgewinkelte Seitenwände (8) mit einer Aussparung (9) für die erste Drehachse (12) und einen nach vorne gerichteten Ansatzt für die zweite Drehachse (6) aufweist.

3. Abschnürvorrichtung nach Anspruch 1 und 2, dadurch gekennzeichnet, daß die Klemmwippe (11) einen geraden Boden mit Seitenwänden (13) für die Aufnahme der ersten und dritten Drehachse (12, 10) und am hinteren Ende eine zum Einrastarm gerichtete Stirnwand (14) aufweist.

4. Abschnürvorrichtung nach einem oder mehreren der vorhegehenden Ansprüche, dadurch gekennzeichnet, daß der Einrastarm (13′) gabelförmig ausgebildet ist und zwei Haken zum Einrasten einer durch zwei seitliche Druckknöpfe (16, 17) betätigbaren und mit Ende des Bandes (5) verbundenen Kappe (15) aufweist.

## Revendications

1. Appareil de ligature pour différentes parties du corps, en particulier pour le bras, pouvant notamment être employé pour provoquer la congestion indispensable dans les cas d'injections ou de prises de sang. Est muni d'un boîtier de fermeture (1) permettant le coulissage d'un ruban (5) formant une boucle (4), et d'une plaque de serrage (11) placée dans le boîtier de fermeture (1) pour assurer alternativement le bloquage ou la libre circulation du ruban; Cette plaque est logée vers le ilieu du boîtier de fermeture (1) et pivote sur un premier axe (12) solidement fixé au boîtier. L'appareil dispose également d'un système de fermeture permettant de bloquer et de débloquer à volonté des extrémités du ruban. Une des caractéristiques de cet appareil consiste dans le fait qu'un deuxième axe (6) est prévu dans la partie avant du boîtier (1) éloignée de la boucle (4); sur cet axe se trouve placée une touche de pression (7) pouvant pivoter et munie à une autre extrémité d'un troisième axe (10) par lequel elle est reliée de manière flexible avec la plaque de serrage (11). De plus, la touche de pression (7) est pourvue à l'autre extrémité d'un système de verrouillage (13) placé en position oblique par rapport au boîtier, afin d'assurer alternativement le serrage et le desserrage de l'une des extrémités du ruban.

2. Appareil de ligature d'après spécification 1, caractérisé par le fait que la touch (7) présente deux parois latérales (8) à angles droits avec une ouverture (9) prévue pour le premier axe (12) et une pièce supplémentaire pour le deuxième axe de rotation (6).

3. Appareil de ligature d'après spécifications 1 et 2, caractérisé par le fait que la plaque de serrage (11) présente un fond plat avec parois latérales (13) pour recevoir le premier et le troisième axe de rotation (12, 10), ainsi qu'une paroi d'about (14) orientée vers la griffe d'encliquetage.

4. Appareil de ligature d'après une ou plusieurs des spécifications exposées ci-dessus, caractérisé par le fait que le système de verrouillage (13) présente une forme fourchue et deux crochets pour l'encliquetage avec un capuchon (15) relié à l'extrémité du ruban (5) et actionnable grâce aux deux touches de pression latérales (16, 17).

## Claims

1. Tourniquet for limbs, especially for the upper arm, used for instance in the compression of blood vessels necessary for injections or withdrawal of blood, with closure casing (1) for pulling through a band (5) forming a loop, and a clamping rocker (11) attached in the closure casing (1) for clamping and releasing the band, which at about the center of the closing casing (1) for clamping and releasing the band, which at about the center of the closure casing (1) is pivoted to a first fulcrum (12) attached to the casing, and with means for the releasable fixing of one band end to the closure which is characterized by that a second fulcrum (6) is provided in the front anti-loop end of the closing casing (1), which a pushbutton (7) is pivoted to, which at the opposite end shows a third fulcrum (10), via which it is pivoted to the clamping rocker (11), and by that the pushbutton (7) shows at the opposite end a lock arm (13') freeing the casing for releasable fixing of the one band end.

2. Tourniquet pursuant to claim 1 characterized by that the key (7) shows two bent sidewalls (8) with a hollow ((9) for reception of the first fulcrum (12) and a front-facing lug for the second fulcrum (6).

3. Tourniquet pursuant to claim 1 and 2 characterized by that the clamping rocker (11) has an even bottom with sidewalls (13) for reception of the first and third fulcrum (12, 10), as well as a front wall (14) facing the lock arm at its rear end.

4. Tourniquet pursuant to one or several of the preceding claims characterized by that the lock arm (13') is of forked shape and shows two hooks for snapping of a cap (15) operated by two lateral push-buttons (16, 17) and connected to the end of the band (5).

Fig.1

15

13'

7

12

6

14

1

3

10

8

9

2

11

5

Fig. 2

4

13'

15

16

17

7

1

5

13'

7

6

10

9   8

Fig. 4

13

14

11

Fig. 5

1   6   12   7

13'

14

3

10

9   8

2

Fig. 3